(19) Europäisches Patentamt
European Patent Office
Office européen des brevets

(11) Veröffentlichungsnummer: **0 324 477 B1**

# (12) EUROPÄISCHE PATENTSCHRIFT

(45) Veröffentlichungstag der Patentschrift: **28.04.93**

(51) Int. Cl.$^5$: **C07F 17/00**, A61K 31/28

(21) Anmeldenummer: **89100486.3**

(22) Anmeldetag: **12.01.89**

Die Akte enthält technische Angaben, die nach dem Eingang der Anmeldung eingereicht wurden und die nicht in dieser Patentschrift enthalten sind.

## (54) Cytostatisch wirksame Titanocen-Derivate und diese Enthaltende Arzneimittel.

(30) Priorität: **13.01.88 DE 3800760**

(43) Veröffentlichungstag der Anmeldung:
**19.07.89 Patentblatt 89/29**

(45) Bekanntmachung des Hinweises auf die Patenterteilung:
**28.04.93 Patentblatt 93/17**

(84) Benannte Vertragsstaaten:
**AT BE CH DE FR GB IT LI LU NL SE**

(56) Entgegenhaltungen:
**EP-A- 0 202 673**
**DE-A- 2 521 923**

**JOURNAL OF ORGANOMETALLIC CHEMISTRY, Band 302, 1986, Seiten 193-200; U. THEWALT et al.: "Kationische Komplexe mit der (Eta5-C5H5)2TiIV-Baugruppe: Darstellung und Struktur von ((Eta5-C5H5)2Ti(bipy))2 + (CF3S = 3-)2 und ((Eta5-C5H5)2Ti(phen))2 + (CF3So3-)2"**

**JOURNAL OF ORGANOMETALLIC CHEMISTRY, Band 342, März 1988, Nr. 2, Seite 167-176; P. KOEPF-MAIER: "Tumorhemmende Cyclopentadienylmetall-Komplexe: experimentelle Untersuchungen zur Ueberprüfung der Cyclopentadien-Hypothese".**

**JOURNAL OF ORGANOMETALLLIC CHEMISTRY, Band 307, 1986, Seiten 319-325; H. KOEPF et al.: "Mono(Eta5-Cyclopentadienyl)-Titan (IV)-und-Zirconium(IV)-Komplexe mit einem oder zwei Toluol-3,4-Dithiolat-Liganden"**

**The Merck Index (1983), Seiten 679 und 6437**

(73) Patentinhaber: **Köpf-Maier, Petra, Prof. Dr.
Bundesring 33
W-1000 Berlin 42(DE)**

(72) Erfinder: **Köpf-Maier, Petra, Prof. Dr.
Bundesring 33
W-1000 Berlin 42(DE)**

(74) Vertreter: **Barz, Peter, Dr. et al
Patentanwälte Dr. V. Schmied-Kowarzik
Dipl.-Ing. G. Dannenberg Dr. P. Weinhold Dr.
D. Gudel Dipl.-Ing. S. Schubert Dr. P. Barz
Siegfriedstrasse 8
W-8000 München 40 (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.5x/3.0.1)

## Beschreibung

Die Erfindung betrifft Titanocen-Derivate, die chemotherapeutisch wirksam sind und insbesondere cytostatische Eigenschaften aufweisen, sowie diese Titanocen-Derivate enthaltende Arzneimittel.

Cytostatisch wirksame Titanocen-Komplexe wurden von der Anmelderin in der DE-C-29 23 334 und der DE-A-35 18 447 beschrieben. Bei weiteren Untersuchungen wurden nun Titanocen-Derivate gefunden, die erhöhte Wasserlöslichkeit aufweisen und eine besseren therapeutischen Index (Quotient aus Toxizität und Aktivität) als Titanocen-dihalogenide besitzen; d. h. sicherer in der therapeutischen Anwendung sind.

Eine Gruppe von erfindungsgemäß verwendeten Titanocen-Derivaten hat die allgemeine Formel I:

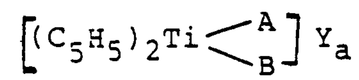

(I)

worin A einen Liganden der Formeln: -N≡C-R, in der R eine Alkylgruppe mit 1 bis 6 Kohlenstoffatomen ist, oder -X-Ar, in der X ein Sauerstoff-, Schwefel- oder Selenatom ist und Ar eine unsubstituierte oder durch Halogen oder $NO_2$ substituierte Phenylgruppe bedeutet, wobei Ar keine unsubstituierte Phenylgruppe sein kann, wenn X ein Schwefelatom ist, bedeutet;
B ein Halogenid bedeutet oder wie der Ligand A definiert ist; oder
A und B zusammen einen zweizähnigen Liganden aus der Gruppe:

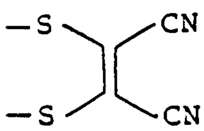 ; 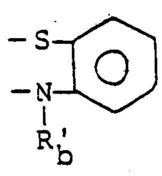 ; 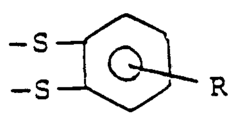 ; 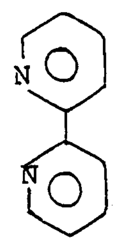 ; oder 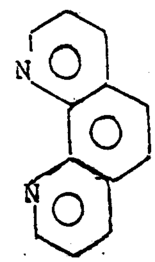

darstellt, wobei R wie oben definiert ist, R′ gleich oder unterschiedlich H oder R bedeutet und b die Zahl 1 oder 2 ist; und
Y ein Anion bedeutet, das dann vorhanden ist, wenn der Titankomplex kationisch geladen ist, wobei a die zum Ladungsausgleich des Gesamtkomplexes erforderliche Zahl bedeutet.

Eine weitere Gruppe von erfindungsgemäß verwendeten Titanocen-Derivaten hat die allgemeine Formel II:

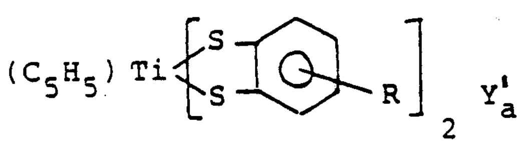

(II)

in der R wie oben definiert ist, Y′ ein Kation bedeutet und a die zum Ladungsausgleich des Gesamtkomplexes erforderliche Zahl ist.

Die vorstehend genannten Alkylgruppen R enthalten 1 bis 6, vorzugsweise 1 bis 4, Kohlenstoffatome. Spezielle Beispiele sind Methyl, Ethyl, n-Propyl, Isopropyl, n-Butyl, tert.-Butyl, Isobutyl, n-Pentyl, n-Hexyl und Cyclohexyl. Die Phenylgruppe Ar kann durch 1, 2, 3, 4 oder 5 Halogenatome, wie Fluor, Chlor oder Brom, oder 1 oder 2 Nitrogruppen substituiert sein. Die Substituenten können in o-, m- oder p-Stellung des Phenylringes stehen.

B ist ein Halogenid-Anion, z. B. Fluorid, Chlorid, Bromid oder Iodid, oder hat eine der Bedeutungen von A. Spezielle Beispiele für das Anion Y sind Halogenid-Anionen, wie Fluorid, Chlorid, Bromid oder Iodid; anionische Komplexe des Typs $[MZ_4]^{n-}$ (n = 1 oder 2) oder $[MZ_6]^{n-}$ (n = 1, 2 oder 3), wobei M = B, Bi, Co, Fe, Ga, Hg, Ru oder Sb und Z = F, Cl, Br oder Phenyl. Hiervon sind besonders bevorzugt $BF_4^-$ , B-

$(C_6H_5)_4^-$, $FeCl_4^-$, $FeBr_4^-$, $BiCl_4^-$, $GaCl_4^-$, $PF_6^-$ und $RuCl_4^-$.

Andere geeignete Anionen Y sind Polyhalogenide, wie $Br_3^-$ und $J_3^-$, Vanadate, Molybdate, Wolframate, Oxyvanadate, Heteropolyanionen, wie Molybdatophosphate, Wolframatophosphate und Wolframatosilicate, sowie Anionen starker organischer Säuren, wie Mono-, Di- oder Trihalogenessigsäure, Cyanessigsäure, Perchlorsäure, Pikrinsäure und Trifluormethansulfonsäure.

Spezielle Beispiele für das Kation $Y'$ sind Ammonium-Kationen des Typs $[NR'_4]^+$, worin die Reste $R'$ gleich oder unterschiedlich sind und die oben genannte Bedeutung haben.

Im folgenden sind repräsentative Beispiele für erfindungsgemäß verwendete Titanocen-Derivate genannt:

I. Titanocen-phenolate, -thiophenolate und -selenophenolate

(1) $(C_5H_5)_2Ti(p\text{-}OC_6H_4NO_2)_2$

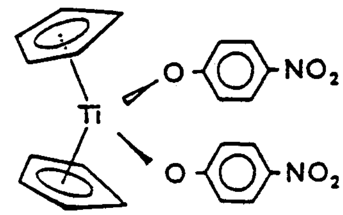

(2) $(C_5H_5)_2Ti(OC_6F_5)_2$

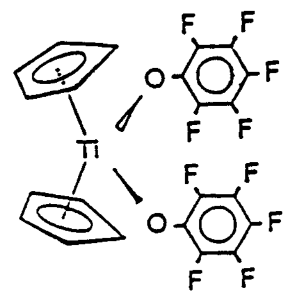

(3) $(C_5H_5)_2Ti(SC_6F_5)_2$

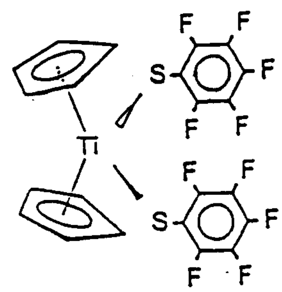

(4) $(C_5H_5)_2TiCl(2,4,6\text{-}OC_6H_2Cl_3)$

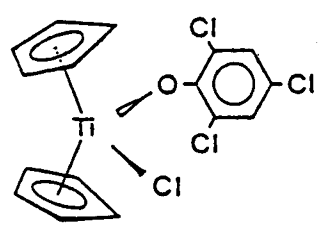

(5) $(C_5H_5)_2TiCl(SeC_6H_5)$

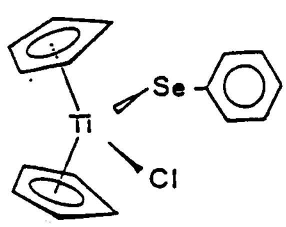

II. Titanocen-dithiolen-Chelat

(6) $(C_5H_5)_2Ti[cis\text{-}1,2\text{-}S_2C_2(CN)_2]$ 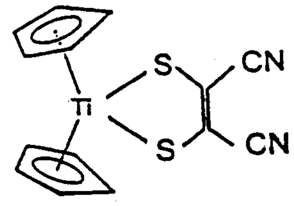

III. Ionische Titanocen-Komplexe

(7) $[(C_5H_5)_2TiCl(NCCH_3)]^+[FeCl_4]^-$ 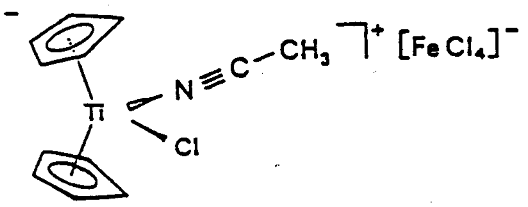

(8) $[(C_5H_5)_2Ti(bipy)]^{2+}[CF_3SO_3]_2^-$ 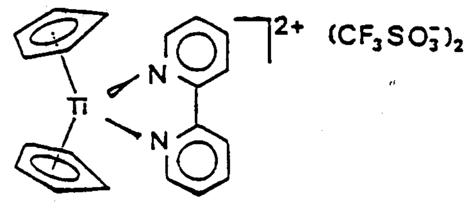

(9) $[(C_5H_5)_2Ti(phen)]^{2+}[CF_3SO_3]_2^-$

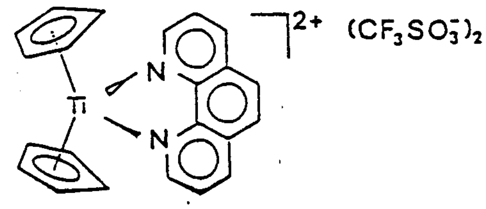

(10) $\{(C_5H_5)_2Ti[o\text{-}S(NHCH_3)C_6H_4]\}^+I^-$

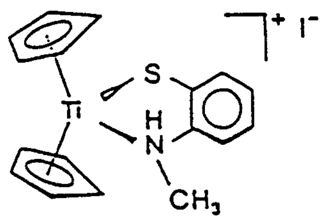

(11) $[(C_5H_5)Ti(1,2,4\text{-}S_2C_6H_3CH_3)_2]^-[N(C_2H_5)_4]^+$

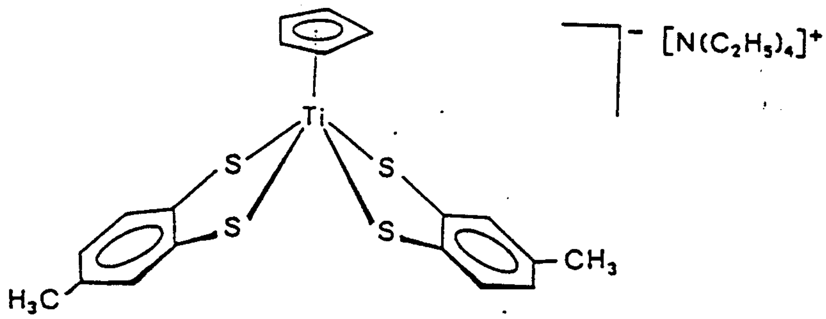

Die vorstehend genannten Titanocen-Derivate sind bekannte Verbindungen, die nach folgenden Literaturvorschriften hergestellt werden können:

Verbindung (1)

Nesmeyanov, A.N., Nogina, O.V., Lazareva, N.A., Dubovitskii, V.A., Lokshin, B.V.: Izv. Akad. Nauk SSSR Ser. Khim. 2482 (1971);
Nesmeyanov, A.N., Nogina, O.V. Dubovitskii, V.A., Kvasov, B.A., Petrovskii, P.V., Lazareva, N.A.: Izv. Akad. Nauk SSSR Ser. Khim. 2729 (1971);

Verbindungen (2) und (3)

Klapötke, T., Köpf, H.: Inorg. Chim. Acta, 133, 115 (1987);

Verbindung (4)

Besançon, J., Camboli, D., Trimaille, B., Colette, M., Tirouflet, J.: Compt. Rend. C 287, 573 (1978);

Verbindung (5)

Köpf, H., Klapötke, T.: Naturforsch. 41b, 971 (1986);

Verbindung (6)

Köpf, H., Schmidt, M.: J. Organomet. Chem. 4, 426 (1965);
Marcellus, C.G., Oakley, R.T., Cordes, A.W., Pennington, W.T.: J. Chem. Soc., Chem. Commun. 1451 (1983);

Verbindung (7)

Meirim, M.G., Neuse, E.W.: Trans. Met. Chem. 9, 337 (1984);
Thewalt, U., Berhalter, K., Neuse, E.W.: Trans. Met. Chem. 10, 393 (1985);

Verbindungen (8) und (9)

Thewalt, U., Berhalter, K.: J. Organomet. Chem. 302, 193 (1986);

Verbindung (10)

Klapötke, T.: Dissertation, Technische Universität Berlin, 1986;

Verbindung (11)

Köpf, H., Klapötke, T.: J. Organomet. Chem. 307, 319 (1986).

Die anderen erfindungsgemäß verwendeten Titanocen-Derivate können nach analogen Verfahren hergestellt werden.

Die erfindungsgemäß verwendeten Titanocen-Derivate zeigen cytostatische Wirkung und eignen sich insbesondere zur Behandlung von soliden Tumoren, z. B. Tumoren des Digestionstraktes, Lungen- und Mamma-Carcinoma.

Aktivität gegenüber Ehrlich-Aszites-Tumor

Weibliche $CF_1$-Mäuse erhalten mittels intraperitonealer Injektion je etwa $6 \cdot 10^6$ Ehrlich-Aszites-Tumorzellen und 24 Stunden später eine einmalige intraperitoneale Substanzgabe (Dosisbereich 20 bis 500 mg/kg) in physiologischer Kochsalzlösung (0,4 ml). Es werden jeweils 5 bis 10 Tiere pro Dosis getestet. Gegebenenfalls kann auf einen pH-Wert von 4 bis 7 gepuffert werden, z. B. mit Natriumhydrogencarbonat oder Tris-(hydroxymethyl)-aminomethan, um eine lokale Irritation am Injektionsort zur vermeiden. In jeder Versuchsreihe wird eine Gruppe von unbehandelten Kontrolltieren, denen 0,4 ml physiologische Kochsalzlösung ohne Substanzgabe intraperitoneal injiziert wurde, mitgeführt.

Die Beurteilung der Tumorentwicklung in den einzelnen Dosisbereichen erfolgt anhand von Gewichtsverlauf und Überlebensdauer. Bei jeder Substanz werden die dosisabhängige Anzahl von Tumortodesfällen, Toxizitätstodesfällen und überlebenden, geheilten Tieren sowie die zugehörige prozentuale Zunahme der mittleren Überlebensdauer bestimmt.

Die bei der Testung der Verbindungen 1 bis 11 erzielten Ergebnisse sind in der folgenden Tabelle 1 wiedergegeben.

Tabelle 1

| Testverbindung | Optimaler Dosisbereich (mg/kg) | Optimale Heilungsrate (%) | Therapeutischer Index | $LD_{50}$ (mg/kg) | $LD_{100}$ (mg/kg) |
|---|---|---|---|---|---|
| (1) | 180-240 | 10 | - | 260 | 360 |
| (2) | 340-360 | 100 | 1,5 | 480 | 540 |
| (3) | 120-180 | 100 | 2,6 | 260 | 320 |
| (4) | 60-140 | 50 | - | 200 | 300 |
| (5) | 80-100 | 80 | - | 150 | 200 |
| (7) | 80-140 | 75 | - | 180 | 220 |
| (8) | 200-220 | 100 | 1,6 | 260 | 300 |
| (9) | 140-220 | 67 | - | 240 | 340 |
| (10) | 200-320 | 100 | 1,7 | 380 | 500 |
| (11) | 40 | 67 | - | 60 | 120 |

Der therapeutische Index ist definiert als der Quotient $LD_{50}/ED_{90}$; bei einer optimalen Heilungsrate < 90 % ist kein Wert angegeben.

Die mit den Verbindungen (2), (3), (5) und (6) erzielten Testergebnisse sind in den Fig. 1 bis 4 graphisch dargestellt.

Aktivität gegenüber Lewis-Lungencarcinom und Colon-38-Adenocarcinom

Für die Testung wurden die Tumoren auf weibliche $B_6D_2F_1$-Mäuse transplantiert. Alle Tiere wurden unter Standardbedingungen (22-23 °C, Leitungswasser und Altromin-Futter ad libitum 12 h Hell-Dunkel-Rhythmus)gehalten.

Für die Tumortransplantation am Tag 0 der Testreihe wurden solide Tumoren aus Donormäusen entfernt, mit der Schere zerkleinert und in kleine Teilchen überführt. Nach dem Suspendieren im zweifachen Volumen Hank-Salzlösung wurden jeweils 0,3 ml subkutan in den Achselbereich geimpft, so daß einzelne solide Tumoren wuchsen.

Die Testverbindung (7) wurde entweder als Einzelinjektion am Tag 1 oder als dreifache Injektionen an den Tagen 1, 3 und 5 oder als fünffache Injektionen an den Tagen 1, 3, 5, 7 und 9 verabfolgt. Die angewandten Dosen und die Tierverteilung sind in Tabelle 2 genannt. Die in einer DMSO-Kochsalz-Mischung (1:9; V:V) gelöste oder suspendierte Substanz wurde stets intraperitoneal injiziert. Die Konzentrationen wurden so gewählt, daß jede Maus ein Gesamtvolumen von 0,4 bis 0,5 ml erhielt. Kontrolltiere erhielten nur eine einzelne, dreifache oder fünffache Injektion des DMSO-Kochsalz-Gemisches (0,5 ml/Tier) ohne Wirkstoffzugabe.

Die Anzahl der Todesfälle wurde täglich aufgezeichnet. Todesfälle, die innerhalb von 7 Tagen (einzelne Injektion), 11 Tagen (dreifache Injektion) oder 15 Tagen (fünffache Injektion) nach der Tumortransplantation auftraten, wurden als Toxizitätstodesfälle aufgrund der Substanztoxizität definiert.

Aus den Tumorgewichten der behandelten Tiere und Kontrolltiere wurden die T/C-Werte folgendermaßen errechnet:

$$T/C\ (\%) = \frac{\text{mittleres Tumorgewicht einer Dosisgruppe x 100}}{\text{mittleres Tumorgewicht der Kontrollgruppe}}$$

In der folgenden Tabelle 2 ist die Antitumoraktivität, ausgedrückt als T/C-Wert, angegeben.

Tabelle 2

| Tumor | Dosis (mg/kg) | T/C (%) |
|---|---|---|
| Lewis-Lungencarcinom | 1 x 70 | 69 |
| | 1 x 80 | 45 |
| | 3 x 40 | 54 |
| | 3 x 50 | 34 |
| | 3 x 60 ($LD_{20}$) | 12 |
| | 5 x 30 | 58 |
| | 5 x 40 | 24 |
| Colon-38-Carcinom | 1 x 60 | 89 |
| | 1 x 70 | 84 |
| | 3 x 40 | 34 |
| | 3 x 50 | 29 |
| | 5 x 30 | 38 |
| | 5 x 40 | 24 |

Zur Krebsbekämpfung können die erfindungsgemäßen Titanocen-Derivate als solche oder als Arzneimittel eingesetzt werden, die mindestens ein Titanocen-Derivat der allgemeinen Formel I oder II neben pharmazeutisch verträglichen Träger-, Verdünnungs- und/oder Hilfsstoffen enthalten. Die pharmazeutische Zubereitung der Wirkstoffe erfolgt vorzugsweise in Form von Einheitsdosen, die auf die jeweilige Applikationsart abgestimmt sind. Eine Einheitsdosis kann z.B. eine Tablette, eine Kapsel, ein Suppositorium oder eine abgemessene Volumenmenge eines Pulvers, eines Granulats oder eine Lösung oder Suspension sein. Unter "Einheitsdosis" wird eine physikalisch bestimmte Einheit verstanden, die eine individuelle Menge des Wirkstoffes in Mischung mit einem geeigneten pharmazeutischen Träger-, Verdünnungs- und/oder Hilfsstoff enthält. Dabei wird die Wirkstoffmenge so gewählt, daß eine oder mehrere Einheiten üblicherweise für eine einzelne therapeutische Verabreichung ausreichen. Die Einheitsdosis kann auch unterteilbar sein, z.B. in Form von gekerbten Tabletten, wenn für eine einzelne therapeutische Verabreichung nur ein Bruchteil, z.B. eine Hälfte oder ein Viertel, der unterteilbaren Einheit benötigt wird. Die Arzneimittel der Erfindung enthalten, wenn sie in Einheitsdosis vorliegen, 1 bis 10 000 mg, vorzugsweise 5 bis 7 500 mg, Wirkstoff.

Die Arzneimittel der Erfindung werden vorzugsweise oral, rectal oder parenteral, z.B. intravenös, subcutan, intramuskulär, intrapleural, intraperitoneal, intrafokal oder perifokal, angewandt. Die therapeutische Verabreichung kann mittels Infusion kontinuierlich über mehrere Stunden oder durch ein- bis mehrmalige Einzelgaben oder Einzelinjektionen erfolgen. Die Verabreichungsfolge und die verabreichte Dosis können in Abhängigkeit von Natur und Stadium der Erkrankung sowie abhängig vom Behandlungsregime, insbesondere von Anzahl und Dosierungshöhe verabreichter Kombinationspräparate, stark variieren. Beispielsweise kann eine Anfangsbehandlung mit täglich 200 bis 800 mg i.v. oder mit Einzeldosen, z.B. 10 bis 40 mg/kg i.v., in entsprechenden Intervallen und eine darauffolgende Dauerbehandlung mit 1 bis 4 Tabletten zu je 50 mg Wirkstoff erfolgen.

Die Arzneimittel bestehen in der Regel aus den erfindungsgemäßen Wirkstoffen und nicht-toxischen, pharmazeutisch annehmbaren Anzneimittelträgern, die als Zumischung in fester, halbfester oder flüssiger Form oder als Umhüllungsmittel, z.B. in Form einer Kapsel, eines Tablettenüberzuges, eines Beutels oder eines anderen Behältnisses für den Wirkstoff in Anwendung kommen. Der Träger kann hierbei z.B. als Vermittler für die Arzneimittelaufnahme durch den Körper, als Formulierungshilfsmittel, Süßungsmittel, Geschmacksmittel, Farbstoff oder Konservierungsmittel dienen.

Für die orale Verabreichung eignen sich z.B. Tabletten, Dragées, Hart- und Weichgelatinekapseln, dispergierbare Pulver, Granulate, wässrige und ölige Suspensionen, Emulsionen, Lösungen und Sirupe.

Tabletten können inerte Verdünnungsmittel, wie Calciumcarbonat, Calciumphosphat, Natriumphosphat oder Lactose; Granulierungs- und Verteilungsmittel, wie Maisstärke oder Alginate; Bindemittel, wie Stärke, Gelatine oder Akaziengummi; und Gleitmittel, wie Aluminium- oder Magnesiumstearat, Talcum oder Siliconöl, enthalten. Gegebenenfalls sind die Tabletten mit einem Überzug versehen, der auch so beschaffen sein kann, daß er eine verzögerte Auflösung und Resorption des Arzneimittels im Gastrointestinaltrakt und damit z.B. bessere Verträglichkeit oder eine längere Wirkungsdauer bewirkt.

Gelatinekapseln können den Wirkstoff im Gemisch mit einem festen (z.B. Calciumcarbonat oder Kaolin) oder öligen (z.B. Oliven-, Erdnuß- oder Paraffinöl) Verdünnungsmittel enthalten.

Als Suspendiermittel eignen sich z.B. Natriumcarboxymethylcellulose, Methylcellulose, Hydroxypropylcellulose, Natriumalginat, Polyvinylpyrrolidon, Traganthgummi oder Akaziengummi; als Dispergier- und

Benetzungsmittel z.B. Polyoxyethylenstearat, Heptadecaethylenoxycetanol, Polyoxyethylensorbitmonooleat, Polyoxyethylensorbitanmonooleat oder Lecithin; als Konservierungsmittel z.B. Methyl- oder Propylhydroxybenzoat; als Geschmacks- und Süßungsmittel z.B. Saccharose, Lactose, Dextrose oder Invertzuckersirup. Ölige Suspensionen können z.B. Erdnuß-, Oliven-, Sesam-, Kokos- oder Paraffinöl sowie Verdickungsmittel, wie Bienenwachs, Hartparaffin oder Cetylalkohol, Süßungsmittel, Geschmacksmittel und/oder Antioxidantien enthalten.

In Wasser dispergierbare Pulver und Granulate enthalten den Wirkstoff im Gemisch mit Dispergier-, Benetzungs- und Suspendiermitteln, z.B. den vorstehend genannten Substanzen und/oder Dimethylsulfoxid, sowie mit Süßungsmitteln, Geschmacksmitteln und/oder Farbstoffen.

Emulsionen können z.B. Oliven-, Erdnuß- oder Paraffinöl neben Emulgatoren, wie Akaziengummi, Traganthgummi, Phosphatiden, Sorbitanmonooleat oder Polyoxyethylensorbitanmonooleat, Süßungsmittel und/oder Geschmacksmitteln enthalten.

Zur rectalen Anwendung eignen sich Suppositorien, die mit Hilfe von bei Rektaltemperatur schmelzenden Bindemitteln hergestellt werden, z.B. Kakaobutter oder Polyethylenglykolen.

Parenteral können die Arzneimittel als sterile isotonische Kochsalzlösungen oder sonstige Lösungen angewandt werden. Um eine gleichmäßige Lösung bzw. Suspendierung zu erzielen, kann ein Lösungsvermittler, wie Dimethylsulfoxid, zugesetzt werden, jedoch ist dies meist nicht erforderlich.

In allen Darreichungsformen können die Arzneimittel der Erfindung außerdem Puffersubstanzen enthalten, z.B. Natriumhydrogencarbonat oder Tris(hydroxymethyl)aminomethan. Neben den erfindungsgemäßen Titanocen-Komplexen können die Arzneimittel einen oder mehrere andere pharmakologisch aktive Bestandteile aus anderen cytostatisch wirksamen Arzneimittelgruppen enthalten, z.B. Alkylantien, Antimetaboliten sowie cytostatische Alkaloide, Antibiotika, Enzyme und Schwermetallverbindungen. Ferner können die Arzneimittel gegebenenfalls immunsuppressiv wirksame Substanzen und Vitamine enthalten. Die genannten Zusatzstoffe können auch in gesonderten pharmazeutischen Zubereitungen als Kombinationspräparate zu den erfindungsgemäßen Wirkstoffen gegeben werden.

Der Wirkstoffgehalt der Arzneimittel beträgt gewöhnlich 0,01 bis 95 Gewichtsprozent, vorzugsweise 0,1 bis 85 Gewichtsprozent, bezogen auf das fertige Arzneimittel.

**Patentansprüche**

**1.** Titanocen-Derivate der allgemeinen Formel I:

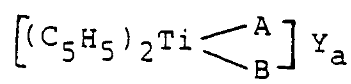 (I)

worin:

A einen Liganden der Formeln: -N≡C-R, in der R eine Alkylgruppe mit 1 bis 6 Kohlenstoffatomen ist, oder -X-Ar in der X ein Sauerstoff-, Schwefel- oder Selenatom ist und Ar eine unsubstituierte oder durch Halogen oder $NO_2$ substituierte Phenylgruppe bedeutet, wobei Ar keine unsubstituierte Phenylgruppe sein kann, wenn X ein Schwefelatom ist, bedeutet;

B ein Halogenid bedeutet oder wie der Ligand A definiert ist; oder

A und B zusammen einen zweizähnigen Liganden aus der Gruppe:

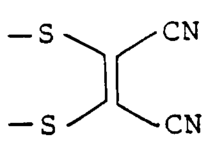 ; 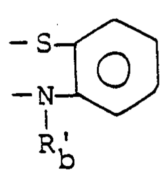 ; 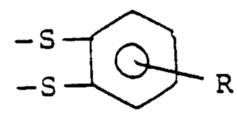 ; 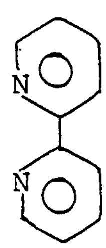 ; oder 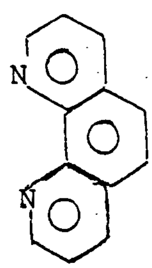

darstellt, wobei R wie oben definiert ist, R′ gleich oder unterschiedlich H oder R bedeutet und b die Zahl 1 oder 2 ist; und

Y ein Anion bedeutet, das dann vorhanden ist, wenn der Titankomplex kationisch geladen ist, wobei a die zum Ladungsausgleich des Gesamtkomplexes erforderliche Zahl bedeutet;
zur Verwendung als Arzneistoffe.

**2.** Titanocen-Derivate der allgemeinen Formel II:

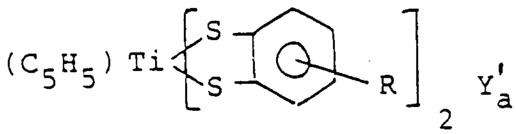

(II)

in der R wie in Anspruch 1 definiert ist, Y′ ein Kation bedeutet und a die zum Ladungsausgleich des Gesamtkomplexes erforderliche Zahl ist;
zur Verwendung als Arzneistoffe.

**3.** Verwendung der Titanocen-Derivate nach Anspruch 1 oder 2 zur Herstellung von Arzneimitteln mit cytostatischer Wirkung.

**4.** Arzneimittel, enthaltend mindestens ein Titanocen-Derivat nach Anspruch 1 oder 2 und übliche Träger- und/oder Hilfsstoffe.

**Claims**

**1.** Titanocene derivatives of the general formula I:

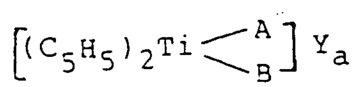

(I)

in which:
A denotes a ligand of the formulae: -N≡C-R, wherein R is an alkyl group having 1 to 6 carbon atoms, or -X-Ar, in which X is an oxygen, sulfur or selenium atom and Ar is a phenyl group which is unsubstituted or substituted by halogen or $NO_2$, it not being possible for Ar to be an unsubstituted phenyl group if X is a sulfur atom;
B denotes a halide or is defined as ligand A; or
A and B together represent a bidentate ligand selected from the group:

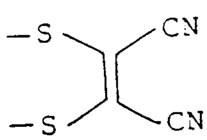 ; 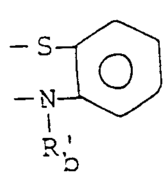 ; 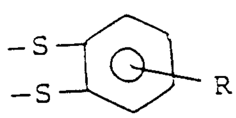 ; 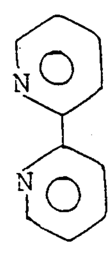 ; or 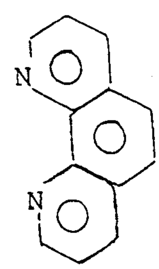

wherein R is as defined above, R', identically or differently, represents H or R, and b is the number 1 or 2; and
Y represents an anion which is present if the titanium complex has a cationic charge, with a denoting the number required for charge compensation of the complete complex;
for use as medicinal substances.

2. Titanocene derivatives of the general formula II:

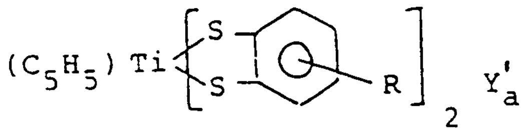

(II)

wherein R is defined as in claim 1, Y' denotes a cation, and a is the number required for charge compensation of the complete complex,
for use as medicinal substances.

3. The use of the titanocene derivatives according to claim 1 or 2 for the preparation of medicaments having cytostatic activity.

4. A pharmaceutical composition containing at least one titanocene derivative according to claim 1 or 2 and conventional carriers and/or excipients.

**Revendications**

1. Dérivés de titanocène de formule générale I :

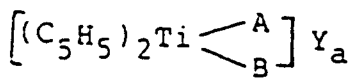

(I)

dans laquelle:
A représente un ligand de formule : -N≡C-R dans laquelle Reest un groupe alkyle ayant 1 à 6 atomes de carbone ou -X-Ar,dans laquelle X représente un atome d'oxygène,de soufre ou de sélénium et Ar représente un groupe phényle non substitué ou substitué par de l'halogène ou par $NO_2$ , Ar ne pouvant pas représenter un groupe phényle non, substitué lorsque X est un atome de soufre ,
B représente un halogénure ou a la même définition que le ligand A ; ou bien
A et B formemnt ensemble un ligand bidenté choisi dans l'ensemble :

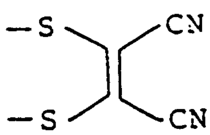 ; 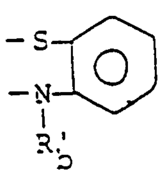 ; 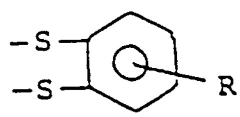 ; 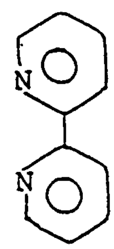 ; ou 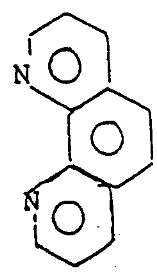

dans lequel R est tel que défini ci-dessus, R' a le même sens que R ou représenteH ,les R' étant identiques ou différents,et b est un nombre valant 1 ou 2; et
Y représente un anion qui est présent lorsque le complexe de titane a une charge cationique et a représente le nombre nécessaire pour l'équilibre des charges du complexe total ;
destinés à servir de médicaments.

**2.** Dérivés de titanocène de formule générale (II)

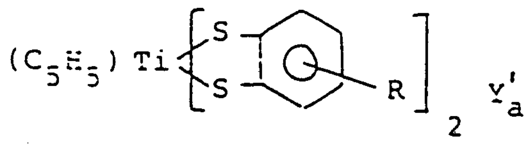

(II)

dans laquelle R a la même définition qu'à la revendication 1 ; Y représente un cation et a est le nombre nécessaire pour l'équilibre des charges du complexe total , destinés à servir de médicaments .

**3.** Utilisation des dérivés de titanocène selon la revendication 1 ou 2 pour préparer des médicaments à action cytostatique .

**4.** Médicament, conte'nant au moins un dérivé de titanocène selon la revendication 1 ou 2 et des excipients et/ou adjuvants usuels .

FIG. 1

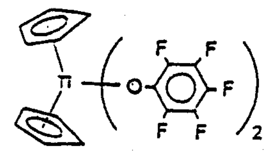

(2)

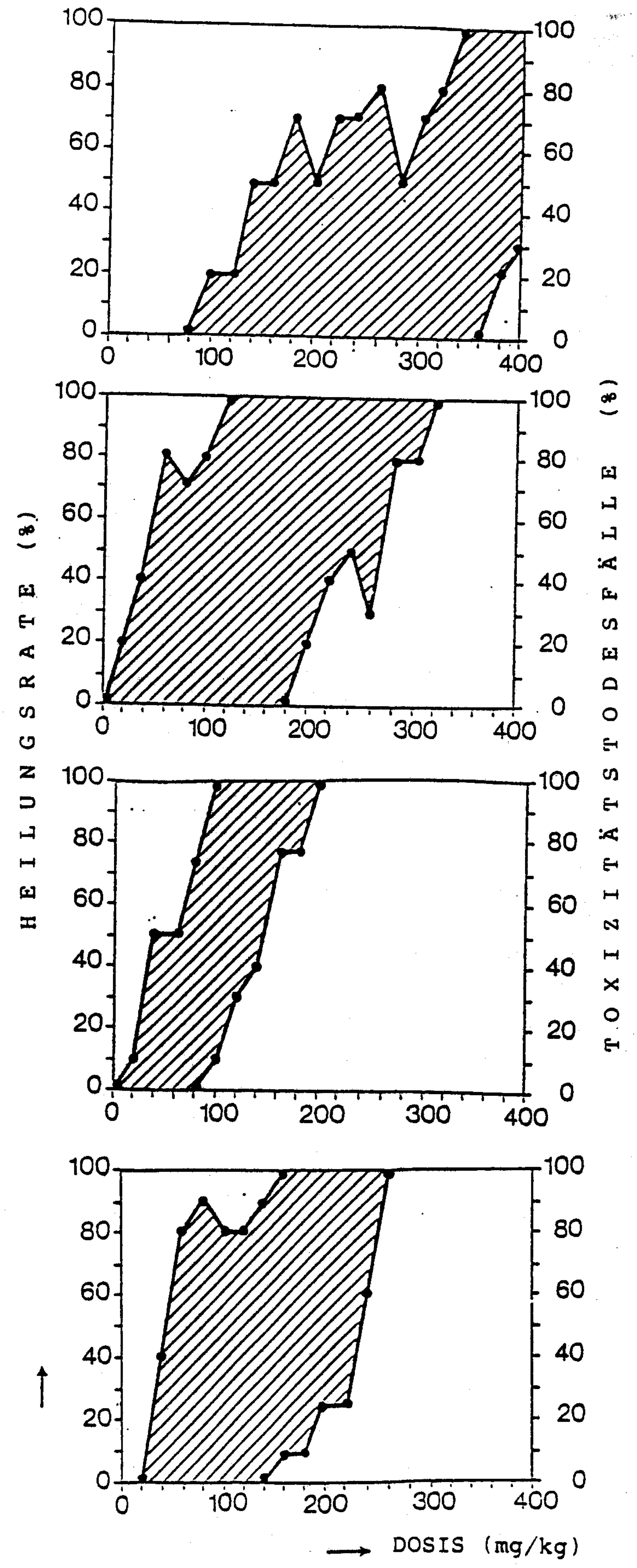


FIG. 2

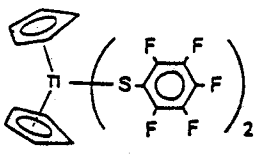

(3)

FIG. 3

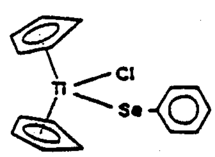

(5)

FIG. 4

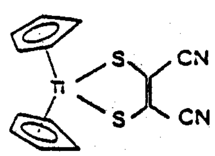

(6)